# EUROPÄISCHE PATENTSCHRIFT

(11) **EP 0 560 809 B1**
(45) Veröffentlichungstag und Bekanntmachung des Hinweises auf die Patenterteilung: **24.08.1994**
(21) Anmeldenummer: 91920447.9
(22) Anmeldetag: 02.12.1991
(51) Int. Cl.: A61K 7/155

(54) **MITTEL ZUM REDUZIEREN DES WACHSTUMS BZW. ENTFERNEN DER HAARE AM MENSCHLICHEN KÖRPER**
AGENT FOR REDUCING THE GROWTH OF, OR REMOVING, HUMAN BODY HAIR
AGENT POUR LA REDUCTION DE LA CROISSANCE OU POUR L'ELIMINATION DES POILS SUR LE CORPS HUMAIN

(30) Priorität: 05.12.1990 DE 4038693
(43) Veröffentlichungstag der Anmeldung: 22.09.1993
(73) Patentinhaber: HEVERHAGEN, Ulrich, D-78303 Radolfzell (DE)
(72) Erfinder: HEVERHAGEN, Ulrich, D-78303 Radolfzell (DE)
(74) Vertreter: Weiss, Peter, Dr. rer.nat.
(86) Internationale Anmeldenummer: DE9100936
(87) Internationale Veröffentlichungsnummer: WO9210164

(56) Entgegenhaltungen:
- EP-A- 0 283 165
- DE-A- 3 243 959
- DE-B- 1 236 728
- DE-B- 2 608 221
- FR-A- 2 588 186
- GB-A- 2 181 349

## Beschreibung

Die Erfindung betrifft ein Mittel entsprechend dem Oberbegriff von Anspruch 1.

Haare sind fadenförmige Gebilde der Außenhaut von Tieren und Menschen und bestehen im wesentlichen aus einer Hornsubstanz. Beim menschlichen Haar sitzt eine zwiebelförmige Haarwurzel im Haarbalg auf einer kleinen blutgefäßreichen Verdickung, der Haarpapille. Von hieraus wird das Haar ernährt und wächst als Haarschaft nach. In den Haarbalg münden kleine Talgdrüsen.

Das Haar besteht aus Hornzellen, die in einer Mark- und einer Rindenschicht angeordnet und von einem Oberhäutchen überzogen sind.

Ein normaler Haarwuchs wird in der Regel toleriert. Störend wirkt sich allerdings in vielen Fällen ein übermäßiger Haarwuchs aus. Hier wird versucht, das Haarwachstum zu reduzieren bzw. ganze Körperpartien von Haaren zu befreien. Hierzu sind verschiedene Verfahren bekannt. Zum einen sind die mechanischen Verfahren zu nennen, wobei beispielsweise mittels einer Pinzette einzelne Haare ausgerissen werden. Dies ist zum einen schmerzhaft, zum anderen sehr zeitaufwendig. Ferner birgt dieses Verfahren die Gefahr in sich, daß eine Wunde entsteht, in welche Bakterien od. dgl. eindringen können.

Des weiteren gibt es die Rasur, wobei hier nur die Haare abgeschnitten werden, sie jedoch nach kurzer Zeit wieder - und meist in verstärktem Umfange - nach-wachsen.

Ferner ist ein Verfahren mit einem heißen oder kalten Wachs bekannt, wobei auch hier die Entfernung nur zeitweise geschieht und vor allem auch Hautirritationen auftreten. Beim kalten Wachs werden die Haare ebenfalls herausgerissen, was wiederum sehr schmerzhaft ist.

Es gibt ferner auf dem Markt auch Creme, Puder, Haarentfernungswässer oder -milch, welche aber allesamt nicht besonders wirkungsvoll und in der Regel sehr aggressiv sind.

Der Erfinder hat sich zum Ziel gesetzt, ein Mittel der o.g. Art zu entwickeln, mittels welchem die Haare wirkungsvoll entfernt werden und dabei vor allem kein Hautangriff stattfindet.

Zur Lösung dieser Aufgabe führt, daß eine Grundsubstanz zum Auftragen auf die Haut, insbesondere demineralisiertes und sterilisiertes Wasser, einen Harnstoff (Urea) enthält.

Dieses Mittel hat vor allem die vorteilhafte Wirkung, daß der Haarwuchs am gesunden Haar reduziert wird. D.h., es findet keine schmerzhafte oder aggressive Behandlung der Haut statt, so daß es hier auch nicht zu Wunden oder Hautirritationen kommt. Es wird lediglich der Haarwuchs gebremst, wobei dieses Reduzieren soweit gehen kann, daß der Haarwuchs gänzlich eingestellt wird.

Harnstoff ist ein Diamid der Kohlensäure und kann auf synthetischem Wege hergestellt werden. Es hat die Wirkung, daß es zelluläre Proteine der Haarwurzel denaturiert und somit die Funktion der Haarwurzel reduziert. Dies wirkt sich dann durch Verminderung des Wachstums aus.

Da Harnstoff andererseits ein körpereigener Stoff ist, schädigt er weder die Haut noch führt er zu Irritationen. Dies gilt für normale Haut. Sollte jedoch eine hyperempfindliche Haut behandelt werden, so hat es sich als ratsam erwiesen, der o.g. Grundsubstanz noch pflanzliche Extrakte mit pflegender Wirkung zuzusetzen. Hierbei bietet sich vor allem Hamamelis, Arnika und Minze an. Diese Stoffe wirken astringierend, tonisch, beruhigend und wundheilend.

Ähnliches gilt auch, wenn der Grundsubstanz tierische Proteine zugesetzt werden. Auch diese wirkt örtlich beruhigend.

Zur Homogenisierung der Zusätze in der Grundsubstanz hat es sich als ratsam erwiesen, der Grundsubstanz ferner ein Hydro- oder Propylenglykol zuzugeben. Propylenglykol ist eine ölige, hygroskopische Flüssigkeit, die durch Hydratisierung von Propylenoxid hergestellt wird. Sie wird in vielen Fällen als Bestandteil von Kosmetika verwendet.

Eine weitere Verbesserung des Mittels ergibt sich, wenn der Grundsubstanz Milchsäure bzw. Lactate, d.h. Salze der Milchsäure, hinzugesetzt werden. Auch sie hat einen Einfluß auf Stoffwechselvorgänge in den Zellen des Haarfollikels.

Ein weiterer Zusatz von Aloe Vera Gel als Feuchtigkeitsspender verstärkt die Wirkung des erfindüngsgemäßen Mittels. Weitere Zusätze, wie Methylparaben, PCA (2-Pyolydon-5-Carbonsäure) dienen vor allem der Homogenisierung und Haltbarmachung des Mittels. Der Bestandteil Bisabolol, dessen Hauptinhalt ein Kamilleextrakt ist, wirkt entzündungshemmend.

In einer bevorzugten Ausführungsform hat das Mittel folgende Zusammensetzung:
68 - 85 Vol% demineralisiertes, sterilisiertes Wasser
0,3 - 1,2 Vol% Harnstoff und/oder
0,2 - 1,0 Vol% tierische Proteine und/oder
0,3 - 1,2 Vol% Pflanzenextrakte und/oder
6 - 15 Vol% Glykol und/oder
0,2 - 0,3 Vol% Milchsäure und/oder
- 1,0 Vol% Aloe Vera Gel und/oder
- 0,2 Vol% Methylparaben und/oder
- 0,1 Vol% PCA und/oder
- 0,1 Vol% Bisabolol.

Im übrigen wird das Mittel in verschiedenen Formen dargereicht. Zum einen soll es als Balsam auf die Haut aufstreichbar sein. Hierbei enthält es demineralisiertes Wasser im unteren Bereich der o.g. Inhaltsangabe, während vor allem zusätzlich noch Stearate und Stearinsäure hinzugegeben ist. Diese bewirken eine Veränderung des Mittels insofern, als es balsamartig wird. Zur Haltbarmachung des Mittels enthält dieses dann noch Isopropyl Myristat. Ferner ist noch Ketylalkohol und/oder Dimethicon und/oder Propylparaben und/oder Triclosan und/oder 2-Brom-2-Nitro-propan-1,3-Diol hinzugegeben.

In einer besonders bevorzugten Ausführungsform enthält das Mittel noch zusätzlich Imidazolidinyl Urea und zwar in einer Menge von 0,2 bis 0,5 Vol%. Dieses spezielle Urea hat sich in der Praxis als äußerst wirkungsvoll auf die Reduzierung des Haarwachstums herausgestellt.

Das Mittel kann ferner noch Hydroxylpropyl Methylzellulose (Gelbildner), Aminosäure und Polyquaternium als kationischen Emulgator enthalten. Es wird außer als Balsam auch als Gel oder in Ampullen angeboten.

Ein unabhängiger Epicutantest nach Jadassohn und Bloch hat ergeben, daß keinerlei Irritationen mit Infiltrationen od. Ödemen oder allergische Reaktionen an Testpersonen, die mit dem Mittel behandelt wurden, auftraten. Dementsprechend wurde das Mittel als "sehr gut hautverträglich" bezeichnet.

Beim Vergleich des Haarwachstums von behandelter und unbehandelter Körperoberfläche konnte festgestellt werden, daß die Längen der nachgewachsenen Haare bei den behandelten Oberflächen deutlich geringer waren, als diejenigen von den unbehandelten Körperoberflächen.

## Patentansprüche

1. Mittel zum Reduzieren des Wachstums bzw. Entfernen der Haare am menschlichen Körper, aus
- demineralisiertem und sterilisiertem Wasser
- einem Harnstoff (Urea)
- tierischen Proteinen, insbesondere hydrolysierten tierischen Proteinen
- pflanzlichen Extrakten, insbesondere von Hamamelis, Arnika, Minze
- Hydro- oder Propylenglykol
- Milchsäuren bzw. Lactaten
- Aloe Vera Gel
- Methylparaben
- PCA (2-Pyolydon-5-Carbonsäure)
- Biasabolol

2. Mittel nach dem Anspruch 1 aus
68 - 85 Vol% demineralisiertem, sterilisiertem Wasser
0,3 - 1,2 Vol% Harnstoff
0,2 - 1,0 Vol% tierischen Proteinen
0,3 - 1,2 Vol% Pflanzenextrakten
6 - 15 Vol% Glykol
0,2 - 0,3 Vol% Milchsäure
- 1,0 Vol% Aloe Vera Gel
- 0,2 Vol% Methylparaben
- 0,1 Vol% PCA und
- 0,1 Vol% Bisabolol

3. Mittel nach Anspruch 2, dadurch gekennzeichnet, daß durch Zugabe von Stearaten und Stearinsäure ein Balsam ausgebildet ist.

4. Mittel nach Anspruch 2 oder 3, dadurch gekennzeichnet, daß ferner
8,0 - 9,0 Vol% Stearate und/oder
1,8 - 2,2 Vol% Stearinsäure und/oder
5,5 - 6,0 Vol% Ketylalkohol und/oder
- 0,5 Vol% Dimethicon und/oder
- 0,1 Vol% Propylparaben und/oder
- 0,1 Vol% Triclosan und/oder
-0,05 Vol% 2-Brom-2-Nitropropan-1,3-Diol enthalten sind.

5. Mittel nach Anspruch 2, dadurch gekennzeichnet, daß zusätzlich 0,2 - 0,5 Vol% Imidazolidinyl Urea enthalten sind.

6. Mittel nach Anspruch 5, dadurch gekennzeichnet, daß ferner etwa 1,5 Vol% Carbomer enthalten sind.

7. Mittel nach Anspruch 6, dadurch gekennzeichnet, daß durch erhöhte Zugabe von Glykol ein Gel ausgebildet ist.

8. Mittel nach Anspruch 5, dadurch gekennzeichnet, daß etwa 0,25 Vol% Hydroxylpropyl Methylcellulose enthalten ist.

9. Mittel nach Anspruch 8, dadurch gekennzeichnet, daß ferner 0,2 - 0,3 Vol% Aminsosäure enthalten ist.

10. Mittel nach Anspruch 9, dadurch gekennzeichnet, daß ferner 1,8 - 2,0 Vol% Aminosäure enthalten ist.

## Claims

1. Agent for reducing the growth of, or removing, human body hair, composed of
- demineralized and sterilized water
- a carbamide (urea)
- animal proteins, in particular hydrolyzed animal proteins
- vegetable extracts, in particular of hamamelis, arnica, mint
- hydro or propylene glycol
- lactic acids and lactates
- aloe vera gel
- methylparabene
- PCA (2-pyolydone-5-carboxylic acid)
- biasabolol

2. Agent according to claim 1 composed of
68 - 85% by volume of demineralized sterilized water
0.3 - 1.2% by volume of carbamide
0.2 - 1.0% by volume of animal proteins
0.3 - 1.2% by volume of vegetable extracts
6 - 15% by volume of glycol
0.2 - 0.3% by volume of lactic acid
- 1.0% by volume of aloe vera gel
- 0.2% by volume of methylparabene
- 0.1% by volume of PCA and
- 0.1% by volume of bisabolol

3. Agent according to claim 2, characterised in that a balsam is formed by addition of stearates and stearic acid.

4. Agent according to claim 2 or 3, characterised in that
8.0 - 9.0% by volume of stearates and/or
1.8 - 2.2% by volume of stearic acid and/or
5.5 - 6.0% by volume of ketyl alcohol and/or
- 0.5% by volume of dimethicone and/or
- 0.1% by volume of propylparabene and/or
- 0.1% by volume of triclosane and/or
-0.05% by volume of 2-bromo-2-nitropropane-1, 3-diol are also contained.

5. Agent according to claim 2, characterised in that 0.2 to 0.5% by volume of imidazolidinyl urea are additionally contained.

6. Agent according to claim 5, characterised in that about 1.5% by volume of carbomer are also contained.

7. Agent according to claim 6, characterised in that a gel is formed by increased addition of glycol.

8. Agent according to claim 5, characterised in that about 0.25% by volume of hydroxypropyl methylcellulose is contained.

9. Agent according to claim 8, characterised in that 0.2 to 0.3% by volume of amino acid is also contained.

10. Agent according to claim 9, characterised in that 1.8 to 2.0% by volume of amino acid is also contained.

## Revendications

1. Agent pour la réduction de la croissance ou l'élimination des poils sur le corps humain à base de :
- eau déminéralisée et stérilisée ;
- une urée (Urea),
des protéines animales, en particulier des protéines animales hydrolysées ;
- des extraits végétaux, en particulier des extraits d'hamamélis, d'arnica et de menthe ;
- de l'hydro ou du propylèneglycol ;
- de l'acide lactique ou des lactates ;
- de l'aloès Vera en gel ;
- du méthylparaben ;
- du PCA (acide de 2e-pyolidone-5 carboxylique) ;
- du bisabolol.

2. Agent selon la revendication 1, à base de :
68-85 % en volume d'eau déminéralisée, stérilisée
0,3 -1,2 % en volume d'urée
0,2-1,0 % en volume de protéines animales
0,3-1,2 % en volume d'extraits végétaux
6-15 % en volume de glycol
0,2-0,3 % en volume d'acide lactique
1 % en volume d'aloès véritable en gel
0,2 % en volume de méthylparaben
0,1 % en volume de PCA et
0,1 % en volume de bisabolol.

3. Agent selon la revendication 2, caractérisé en ce qu'un produit balsanique est formé par addition de stéarates et d'acide stéarique.

4. Agent selon la revendication 2 ou 3, caractérisé en ce qu'il contient en outre :
8,0 à 9 % en volume de stéarate, et/ou
1,8 à 2,2 % en volume d'acide stéarique, et/ou
5,5 à 6,0 % en volume d'alcool cétylique, et/ou
0,5 % en volume de diméthicone, et/ou
0,1 % en volume de propylparaben, et/ou
0,1 % en volume de triclosan, et/ou
0,05 % de 2-bromo-2-nitropropane-1,3-diol.

5. Agent selon la revendication 2, caractérisé en ce qu'il contient en supplément de 0,2 à 0,5 % en volume d'imidazolidinyl urée.

6. Agent selon la revendication 5, caractérisé en ce qu'il contient en outre, environ 1,5 % en volume de carbomère.

7. Agent selon la revendication 6, caractérisé en ce que par addition accrue de glycol, un gel est formé.

8. Agent selon la revendication 5, caractérisé en ce qu'il contient environ 0,25 % en volume d'hydroxylpropyl méthylcellulose.

9. Agent selon la revendication 8, caractérisé en ce qu'il contient en outre 0,2 à 0,3 % en volume d'acide aminé.

10. Agent selon la revendication 9, caractérisé en ce qu'il contient en outre 1,8 à 2,0 % en volume d'acide aminé.
